# EUROPEAN PATENT SPECIFICATION

(11) **EP 3 862 704 B1**
(45) Date of publication and mention of the grant of the patent: **22.03.2023**
(21) Application number: 19888565.9
(22) Date of filing: 30.10.2019
(51) Int. Cl.: F25D 17/04, F25D 21/14, A61L 9/22, F25D 17/06

(54) **AIR-COOLED REFRIGERATOR**
LUFTGEKÜHLTER KÜHLSCHRANK
RÉFRIGÉRATEUR À REFROIDISSEMENT PAR AIR

(30) Priority: 30.11.2018 CN 201811458779
(43) Date of publication of application: 11.08.2021
(73) Proprietor: Haier Smart Home Co., Ltd., Qingdao, Shandong 266101 (CN); Qingdao Haier Refrigerator Co., Ltd., Laoshan District Qingdao Shandong 266101 (CN)
(72) Inventor: ZHU, Xiaobing, Qingdao, Shandong 266101 (CN); FEI, Bin, Qingdao, Shandong 266101 (CN); WANG, Ming, Qingdao, Shandong 266101 (CN); JIANG, Bo, Qingdao, Shandong 266101 (CN); CHENG, Xueli, Qingdao, Shandong 266101 (CN)
(74) Representative: Ziebig Hengelhaupt Intellectual Property Attorneys Patentanwaltsanwaltskanzlei PartGmbB
(86) International application number: PCT/CN2019/114479
(87) International publication number: WO 2020/108221

(56) References cited:
- WO-A1-2010/024078
- CN-A- 103 119 385
- JP-A- 2003 042 645
- JP-A- 2005 042 989
- KR-A- 20000 041 599
- KR-A- 20070 052 507
- US-A1- 2012 181 911

## Description

### FIELD OF THE INVENTION

The present invention relates to the technical field of refrigeration equipment, and particularly relates to an air-cooled refrigerator.

### BACKGROUND OF THE INVENTION

A function of a refrigerator is to provide a low-temperature environment to prolong storage time of articles. However, in the use process of the refrigerator, food and its attached dirt in the refrigerator may suffer from biochemical effects such as oxidative decomposition, thereby generating an unpleasant odor in the refrigerator. Taint of odors may occur among the odors inside the refrigerator and the food stored inside the refrigerator, which seriously affects the use experience of a user. Additionally, the food stored in a storage space of the refrigerator, especially the food stored in a refrigerating chamber will decay due to bacteria after long-time storage, and thus food safety will be influenced.

In order to remove the bacteria and the odors in the refrigerator, people initially installed an adsorption device in the storage space of the refrigerator, but the adsorption device did not achieve any effect on the bacteria on a surface of an inner liner of the refrigerator and on the food in the storage space, and it only absorbed odor generated by microbes, and did not take any effect on food storage. Some refrigerators are also provided with sterilization devices; sterilization devices are all directly placed in positions of refrigerator compartments, and the diffusion of sterilization substances is slow and non-uniform.

JP 2003 042645 A discloses a refrigerator with a negative ion generator. The negative ion generator comprises a high-voltage electrode device for performing corona discharge. In JP 2005 042989 A a single electrode arrangement for creation of ozone molecules in a refrigerator is described.

### BRIEF DESCRIPTION OF THE INVENTION

An objective of the present invention is to provide an air-cooled refrigerator, in which a deodorization and sterilization substance is diffused at an increased speed and is distributed uniformly in a storage space.

Particularly, the present invention provides an air-cooled refrigerator, including a cabinet, an evaporator and an air duct assembly. A storage space is defined inside the cabinet; the evaporator and the air duct assembly are disposed inside the cabinet; and air supplied to the storage space is cooled by the evaporator; the air duct assembly is provided with at least one air supply duct, and is configured to supply the air cooled by the evaporator to the storage space in a controlled manner.

An accommodation space is disposed inside the cabinet or the air duct assembly, and the accommodation space is located outside each of the at least one air supply duct.

The air-cooled refrigerator further includes a deodorization and sterilization apparatus, and the deodorization and sterilization apparatus is provided with:
a control module, disposed in the accommodation space;
an ion generation module, disposed inside one of the at least one air supply duct; and
a connecting cable, electrically connected to the control module and the ion generation module, wherein the ion generation module includes an ionization head and a base, wherein the ionization head is provided with a first electrode and a second electrode connected with the connecting cable and wherein a discharging gap is formed between the first electrode and the second electrode, and the discharging gap is configured to be broken down by a first voltage so that the surrounding air is excited to be ionized.

The control module is provided with a control circuit module, and the control circuit module includes a power supply input interface, a boost circuit and a high-voltage output interface; the power supply input interface is configured to be connected with a direct current power supply provided externally; the boost circuit is configured to convert the direct current power supply into a first voltage for generating a deodorization and sterilization substance and a second voltage for heating in a controlled manner; and the high-voltage output interface is configured to output the first voltage or the second voltage, wherein the first voltage is higher than the second voltage;
the connecting cable is connected to the high-voltage output interface and the ion generation module; and
the ion generation module is configured to be excited by the first voltage to ionize air so as to generate the deodorization and sterilization substance, and to generate heat at the second voltage so as to eliminate condensation.

Optionally, the deodorization and sterilization substance generated by the ion generation module includes at least one of singlet active oxygen, oxygen anion, hydroxyl radical, ozone and hydrogen peroxide.

Optionally, the at least one air supply duct includes a first air duct configured to supply air to an upper portion of the storage space;
a lower portion of the storage space is provided with an air return port; and
the ion generation module is disposed in the first air duct.

Optionally, the air duct assembly further includes an air duct foam and an air duct cover plate;
at least one of the at least one air supply duct is defined at a back side of the air duct foam;
the accommodation space and a cable groove communicating the accommodation space and the first air duct are disposed in a front side of the air duct foam; and the connecting cable is installed in the cable groove; and
the air duct cover plate is installed at the front side of the air duct foam.

Since the air-cooled refrigerator of the present invention is provided with the deodorization and sterilization apparatus, and the ion generation module of the deodorization and sterilization apparatus is disposed in the air supply duct, so that a generated deodorization and sterilization substance can enter the storage space along with cooled air, and the deodorization and sterilization substance is diffused at a high speed, and is distributed uniformly along with an airflow in the storage space.

Particularly, the control module and the ion generation module are separately disposed, and the control module is disposed at the outer side of the air duct, which can prevent damage and failure of the control module caused by the cooled air or a high-humidity environment in the refrigerator, and particularly preventing moisture in the cooled air from entering the control module. The control module does not occupy a space of the air supply duct, so that the air supply of the air supply duct is smooth.

Further, the deodorization and sterilization substance can be controlled to be released and stopped by opening and closing the air supply duct.

According to the following detailed description of specific embodiments of the present invention in conjunction with the drawings, those skilled in the art will more clearly understand the above and other objectives, advantages and features of the present invention.

### BRIEF DESCRIPTION OF THE DRAWINGS

Some specific embodiments of the present invention are described in detail below with reference to the drawings by way of example and not limitation. The same reference numerals in the drawings indicate the same or similar components or parts. Those skilled in the art should understand that these drawings are not necessarily drawn in scale. In the drawings:
Figure 1 is a schematic partial structure diagram of an air-cooled refrigerator according to an embodiment of the present invention.
Figure 2 is a schematic structure diagram of an air duct assembly in the air-cooled refrigerator shown in Figure 1.
Figure 3 is a schematic structure diagram of the air duct assembly shown in Figure 2.
Figure 4 is a schematic exploded view of the air duct assembly shown in Figure 2.
Figure 5 is a schematic structure diagram of a deodorization and sterilization apparatus in the air-cooled refrigerator shown in Figure 1.
Figure 6 is a schematic structure diagram in another perspective of the deodorization and sterilization apparatus shown in Figure 5.
Figure 7 is a schematic diagram of airflow flowing in the air duct assembly and a storage space in the air-cooled refrigerator shown in Figure 1.
Figure 8 is a schematic exploded view of the deodorization and sterilization apparatus shown in Figure 5.
Figure 9 is a schematic partial structure diagram of the deodorization and sterilization apparatus shown in Figure 5.
Figure 10 is a schematic cross-sectional view of the deodorization and sterilization apparatus shown in Figure 5.
Figure 11 is another schematic cross-sectional view of the deodorization and sterilization apparatus shown in Figure 5.
Figure 12 is a schematic structure block diagram of the air-cooled refrigerator according to an embodiment of the present invention.
Figure 13 is a schematic structure block diagram of a control circuit module of the air-cooled refrigerator according to an embodiment of the present invention.

### DETAILED DESCRIPTION

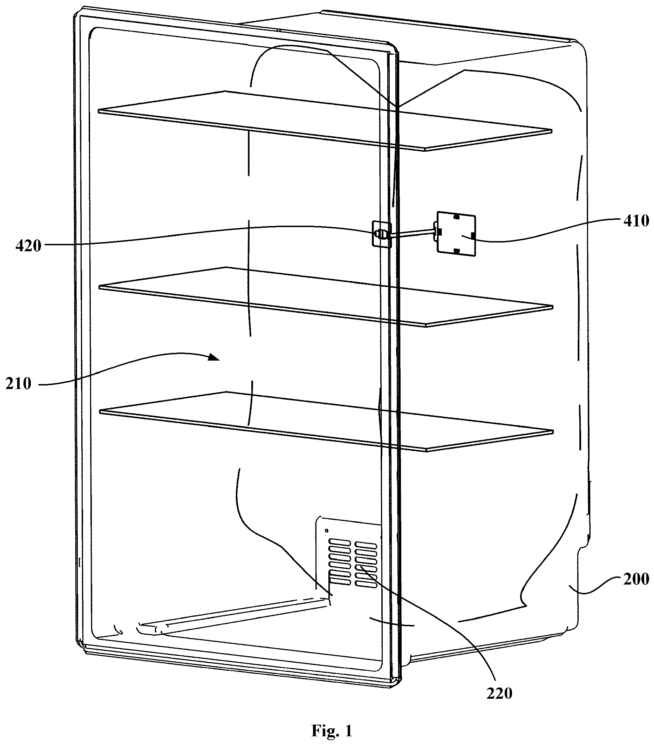

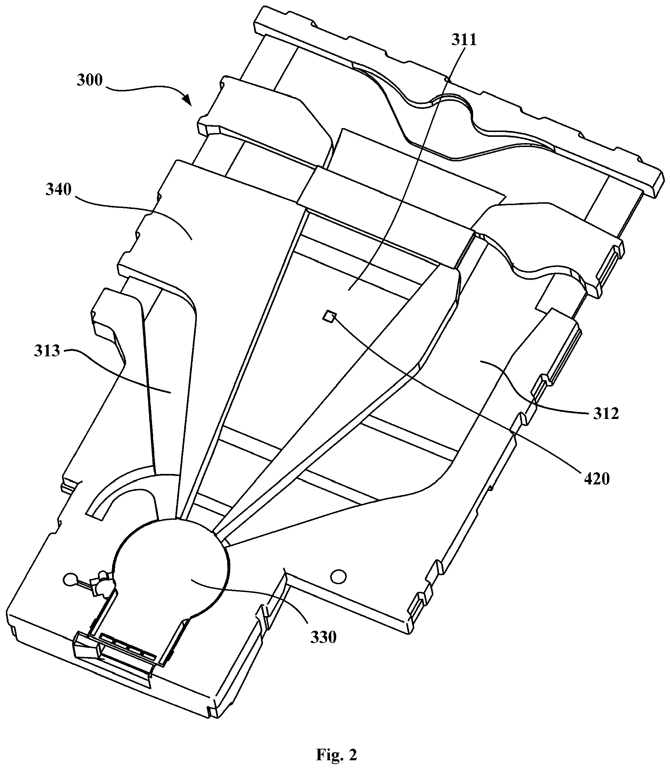

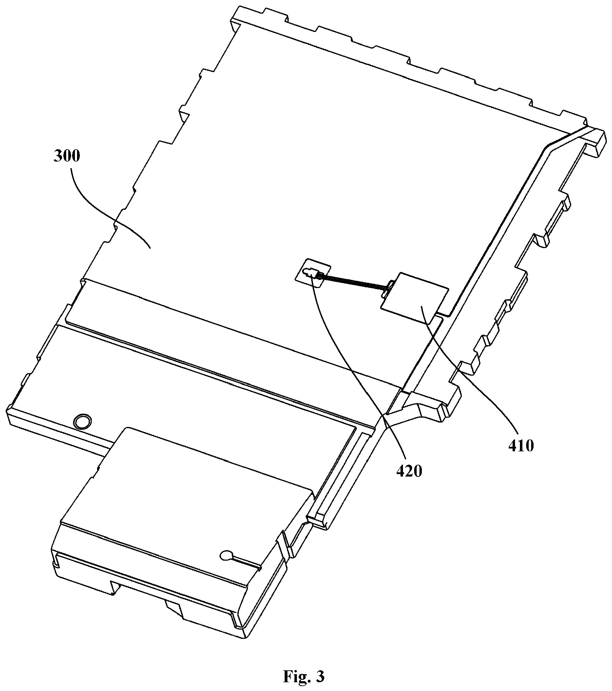

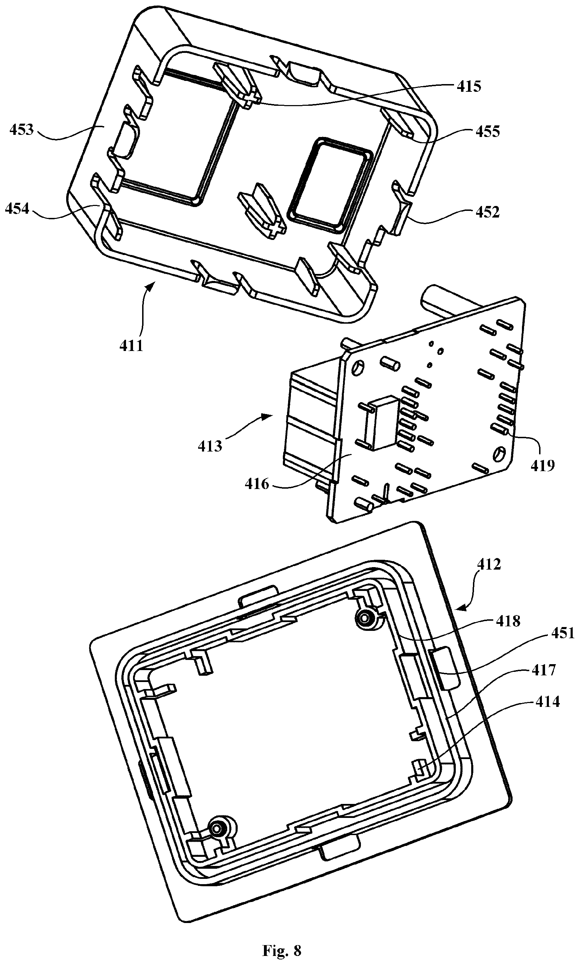

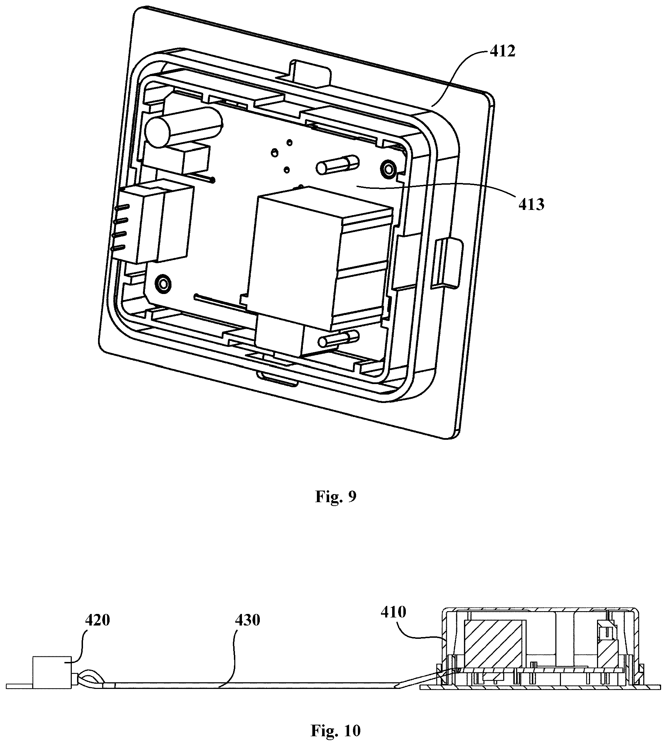

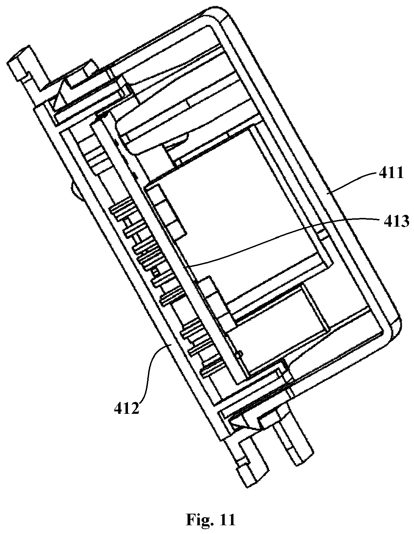

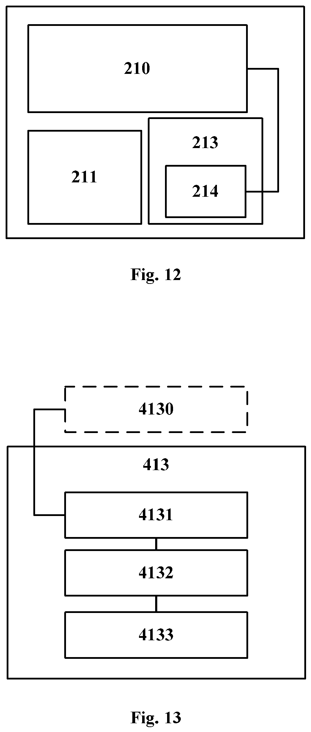

Figure 1 is a schematic partial structure diagram of an air-cooled refrigerator according to an embodiment of the present invention. As shown in Figure 1, and referring to Figure 2 to Figure 12, the embodiment of the present invention provides an air-cooled refrigerator. The air-cooled refrigerator includes a cabinet 200. The cabinet 200 may include a shell made of steel plates and provided with an opening at a front side, an inner liner made of synthetic resin, disposed in an inner space of the shell and provided with an opening at a front side, and a heat insulation material made of polyurethane foam, which is formed through filling and foaming in a gap between the shell and the inner liner. A storage compartment configured to store food and the like is formed in the cabinet 200. According to a storage temperature and use, an inside of the storage compartment is separated into a storage space 210 and at least one other collection compartment, for example, the storage space 210 is a first storage compartment, and the other collection compartment may be a second storage compartment 211. The first storage compartment may be a refrigeration space, a freezing space and/or a variable-temperature compartment, etc. The second storage compartment 211 may also be a refrigeration space, a freezing space and/or a variable-temperature chamber, etc. The first storage compartment is preferably a refrigeration space, and the second storage compartment 211 is preferably a freezing space and disposed at a lower side of the first storage compartment. The at least one other collection compartment further includes a cooling chamber 213, and the cooling chamber 213 is disposed at a back side of the second storage compartment 211.

As it can be understood by those skilled in the art, the air-cooled refrigerator according to the embodiment of the present invention, as an existing air-cooled refrigerator, may further include a compressor, includes an evaporator 214, and and may include a condenser, a throttling element, etc. The evaporator is connected to the compressor, the condenser and the throttling element through a refrigerant pipeline to form a refrigeration cycle loop. The temperature is lowered when the compressor is started, so that air passing through the evaporator is cooled. The air cooled through the evaporator is supplied into respective compartments, so that the temperature in respective compartments is kept in a corresponding set temperature range. The evaporator 214 is disposed in the cooling chamber 213.

The air-cooled refrigerator further includes an air duct assembly 300 configured to transmit the air cooled through the evaporator to the storage space 210, and may further include an air return air duct configured to convey air flowing out from the storage space 210 to the evaporator to be cooled, and an air blower configured to drive the air cooled through the evaporator to flow toward the storage space 210. The air duct assembly 300 is provided with at least one air supply duct 310.

Particularly, an accommodation space 320 is disposed inside the cabinet 200 or the air duct assembly 300, and the accommodation space 320 is located outside each air supply duct 310. The air-cooled refrigerator further includes a deodorization and sterilization apparatus 400. The deodorization and sterilization apparatus 400 is provided with a control module 410, an ion generation module 420 and a connecting cable 430. The control module 410 is disposed in the accommodation space 320. The ion generation module 420 is disposed inside one air supply duct 310. The connecting cable 430 is electrically connected to the control module 410 and the ion generation module 420. The control module 410 controls the ion generation module 420 through the connecting cable 430. The ion generation module 420 may ionize air to generate a deodorization and sterilization substance.

The deodorization and sterilization substance may enter the storage space 210 along with the cooled air. The deodorization and sterilization substance is diffused at a high speed, and is distributed uniformly along with an airflow in the storage space 210. The control module 410 and the ion generation module 420 are separately disposed, and the control module 410 is disposed at the outer side of the air duct, which may prevent damage and failure of the control module 410 caused by the cooled air or a high-humidity environment in the refrigerator, and particularly preventing moisture in the cooled air from entering the control module 410. The control module 410 does not occupy a space of the air supply duct 310, so that the air supply of the air supply duct 310 is smooth. Further, the deodorization and sterilization substance may be controlled to be released and stopped by opening and closing the air supply duct 310. That is, after the corresponding air supply duct 310 is closed, no flowing airflow exists, and the deodorization and sterilization substance does not perform sterilization along with flowing of the airflow, which may be equivalent to stopping the release of the deodorization and sterilization substance. Further, when the corresponding air supply duct 310 is closed, the control module 410 may also make the ion generation module 420 stop working.

In some embodiments of the present invention, the deodorization and sterilization substance generated by the ion generation module 420 includes at least one of singlet active oxygen, oxygen anion, hydroxyl radical, ozone and hydrogen peroxide.

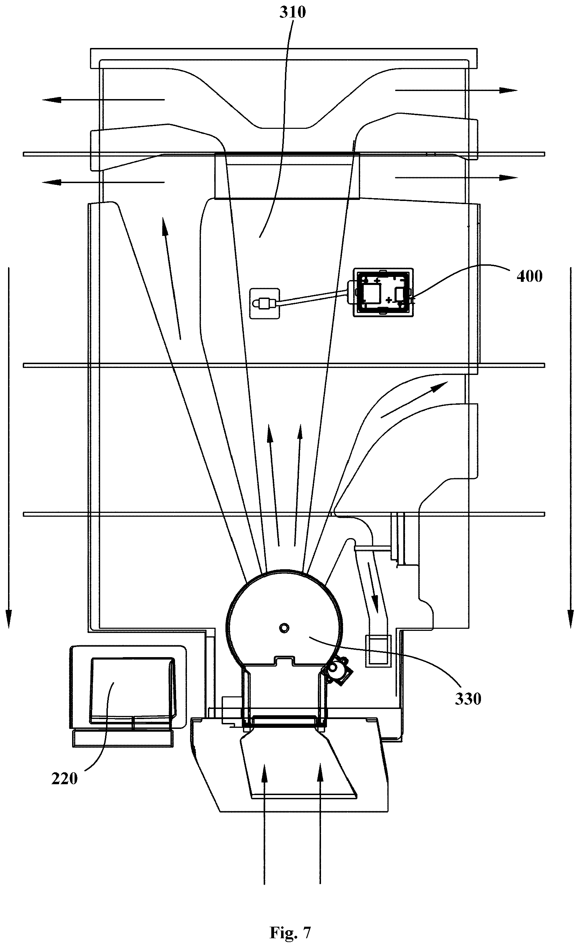

In some embodiments of the present invention, the at least one air supply duct 310 includes a first air duct 311 configured to supply air to an upper portion of the storage space 210. A lower portion of the storage space 210 is provided with an air return port 220. The ion generation module 420 is disposed in the first air duct 311. As shown in Figure 1 and Figure 7, through such an arrangement, the deodorization and sterilization substance may be quickly and uniformly distributed in the storage space 210.

Preferably, the first air duct 311 is provided with two first air supply ports disposed at two sides of the upper portion of the storage space 210 and enabling cooled air to flow toward two sides of the storage space 210. That is, each of the first air supply ports faces one side wall of the storage space 210, and it may either face the corresponding side wall directly, or face the corresponding side wall in a forward inclined manner, or face the corresponding side wall in a downward inclined manner.

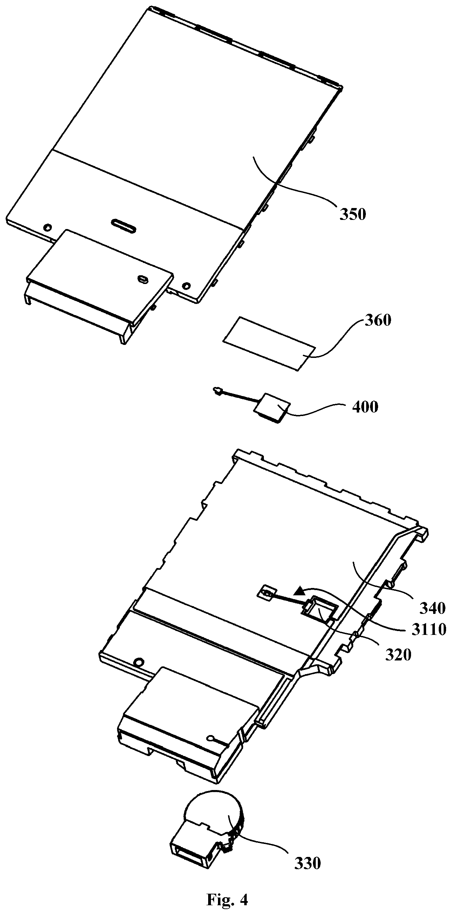

In some embodiments of the present invention, as shown in Figure 2 and Figure 4, the at least one air supply duct 310 refers to a plurality of air supply ducts, for example, the at least one air supply duct 310 also includes a second air duct 312 and a third air duct 313. The second air duct 312 may be disposed at one side of the first air duct 311, and an upper end of the second air duct is connected with two second air supply ports, and each of the second air supply ports is located at a lower side of the first air supply port. In order to facilitate the flowing of the airflow, the second air supply port located at the other side of the first air duct 311 may connect with the second air duct through a bridging air pipe. The third air duct 313 may be located at the other side of the first air duct 311. The third air duct 313 is configured to convey an airflow toward the bottom of the storage space 210. A branched air supply device 330 is disposed at an air inlet of the air duct assembly 300. The branched air supply device 330 is provided with a plurality of air outlets, and each of the air outlets connects with one air supply duct 310.

In some embodiments of the present invention, the air duct assembly 300 further includes an air duct foam 340 and an air duct cover plate 350. At least one air supply duct 310 is defined at a back side of the air duct foam 340. The air supply duct 310 may be defined between the air duct foam 340 and a back wall surface of the inner liner. Alternatively, the air duct assembly 300 further includes an air duct bottom plate installed at a back wall surface of the corresponding inner liner. The air duct foam 340 is installed at the air duct bottom plate, and defines at least one air supply duct 310 together with the air duct bottom plate.

The accommodation space 320 and a cable groove 3110 connecting the accommodation space 320 and the first duct 311 are disposed at a front side of the air duct foam 340. The connecting cable 430 is installed in the cable groove 3110. The air duct cover plate 350 is installed at the front side of the air duct foam 340. Specifically, the accommodation space 320 may be located at one side of the first air duct 311 far away from the second air duct, and is located at an upper side of the first air duct 311, so as to sufficiently and reasonably lay out the air duct assembly 300. The cable groove 3110 is disposed in an inclined manner, and the ion generation module 420 is disposed at a lower side of the control module 410, so as to prevent condensed water and the like from entering the control module 410 along the cable groove 3110 as much as possible.

Preferably, a water blocking device 360 is disposed at front sides of the accommodation space 320 and the cable groove 3110. The water blocking device 360 is located between the air duct cover plate 350 and the control module 410. The water blocking device 360 is PE foam. During installation, the PE foam may be pasted on back surfaces of the control module 410 and the ion generation module 420 for pre-fixation, and then, the air duct cover plate 350 may be installed. The PE foam may also be pasted on all outer surfaces of the control module 410.

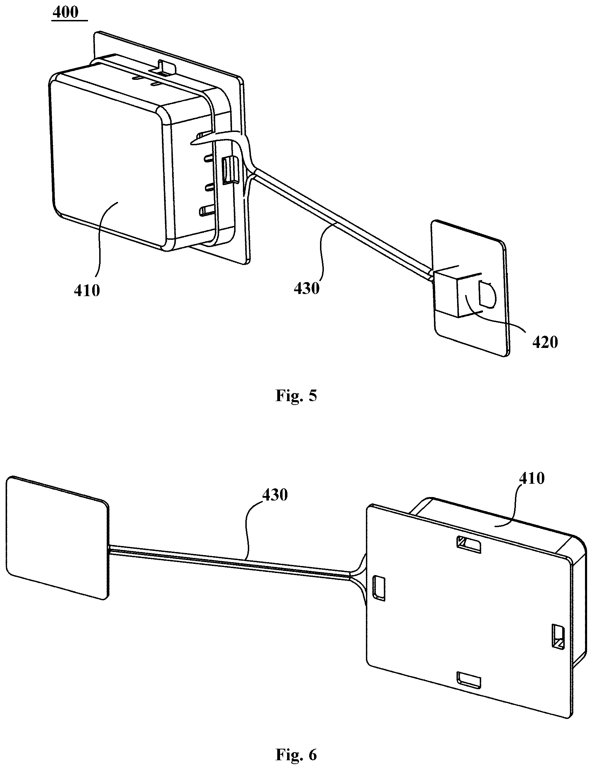

In some embodiments of the present invention, as shown in Figure 5, Figure 6, and Figure 8 to Figure 13, the control module 410 includes a box body and a control circuit module 413 disposed in the box body. The control circuit module 413 is electrically connected to the ion generation module 420 through the connecting cable 430. The box body includes a first box body 411 and a second box body 412 clamped to the first box body 411. A lug boss 414 is disposed at an inner side of the second box body 412, and a support post 415 is disposed at an inner side of the first box body 411. The control circuit module 413 includes a circuit board 416. One side of the circuit board 416 is in contact with and abuts against the lug boss 414, and the other side is in contact with and abuts against the support post 415.

In some embodiments of the present invention, the second box body 412 includes a first base plate, a first peripheral wall 417 and a second peripheral wall 418. The first peripheral wall 417 and the second peripheral wall 418 extend out from the same side of the first base plate, and the first peripheral wall 417 is located at an outer side of the second peripheral wall 418. A plurality of clamping holes 451 are formed in the first peripheral wall 417. A plurality of buckles 452 are formed on the peripheral wall of the first box body 411. Additionally, the peripheral wall of the first box body 411 is inserted between the first peripheral wall 417 and the second peripheral wall 418. Each buckle 452 is inserted into one clamping hole 451. Further, the first box body 411 includes a second base plate. Additionally, the peripheral wall of the first box body 411 includes a peripheral wall base portion 453 extending out from the edge of the second base plate toward one side of the second base plate, and a plurality of peripheral wall sections 454 extending out from a tail end of the peripheral wall base portion 453 and disposed at intervals in a direction along the peripheral wall base portion 453. Each buckle 452 is connected to a tail end of the peripheral wall base portion 453, is disposed between two adjacent peripheral wall sections 454, and is disposed at intervals with the corresponding peripheral wall sections 454. The number of notches between the two adjacent peripheral wall sections 454 is greater than the number of the buckles 452, and the notches may be configured to allow the connecting cable 430 to pass through. That is, the notches between the two adjacent peripheral wall sections 454 include a plurality of buckle notches and at least one threading notch. Each buckle 452 is connected to the tail end of the peripheral wall base portion 453, is disposed in one buckle notch, and is disposed at intervals with the corresponding peripheral wall sections 454. The connecting cable 430 passes through the threading notches.

Demising grooves are disposed in positions corresponding to the clamping holes 451 on the second peripheral wall 418. Communication holes are formed in positions near the clamping holes 451 on the first base plate, so that water between the first peripheral wall 417 and the second peripheral wall 418 may flow out. Additionally, the peripheral wall of the first box body 411 is in contact with and abuts against the first peripheral wall 417. A length of the clamping hole 451 in the peripheral direction of the first peripheral wall 417 is greater than a length of the corresponding buckle 452. A support plate 455 is disposed at an inner side of a peripheral wall of the first box body 411. The lower end of the support plate 455 is in contact with and abuts against the second peripheral wall 418. Such an arrangement facilitates condensed water and the like in the box body to flow out. During installation, the control module 410 may ensure at least a 7-degree angle with a horizontal direction, and may remove water tension, thereby preventing water drops from entering the middle of the circuit board 416. A waterproof groove may be formed by the first peripheral wall 417, the second peripheral wall 418 and the peripheral wall of the first box body 411 to prevent water from entering the circuit board 416.

In some embodiments of the present invention, a plurality of heat radiation posts 419 are disposed at one side of the circuit board 416 facing the lug boss 414. The circuit board 416 is also provided with a power supply input interface 4131, a boost circuit 4132 and a high-voltage output interface 4133. The power supply input interface 4131 is configured to be connected with an externally provided direct current power supply 4130. The boost circuit 4132 is configured to convert the direct current power supply 4130 into a first voltage for generating a deodorization and sterilization substance and a second voltage for heating in a controlled manner. The high-voltage output interface 4133 is configured to output the first voltage or the second voltage. The first voltage is higher than the second voltage. The connecting cable 430 is connected to the high-voltage output interface 4133 and the ion generation module 420. The ion generation module 420 is configured to be excited by the first voltage to ionize air so as to generate the deodorization and sterilization substance, and to generate heat at the second voltage so as to eliminate condensation.

The ion generation module 420 includes an ionization head and a base. The ionization head is provided with a first electrode and a second electrode connected with the connecting cable 430. A discharging gap is formed between the first electrode and the second electrode, and the discharging gap is configured to be broken down by the first voltage so that the surrounding air is excited to be ionized. Additionally, under the second voltage, the first electrode and the second electrode are kept isolated and respectively generate heat. The base forms an installing cavity configured to install the ionization head. One side of the installing cavity is provided with an opening. Additionally, the ionization head is installed to enable the discharging gap to be exposed to the opening.

In some embodiments of the present invention, the control circuit module 413 further includes a voltage feedback port, a controlled end and an inverter. The voltage feedback port is connected with the boost circuit 4132, and is configured to output a feedback voltage corresponding to the first voltage or the second voltage, so as to indicate a boosting state of the boost circuit 4132. The controlled end is connected with the boost circuit 4132, and is configured to receive an external control signal (the control signal may be a control signal sent by a main control panel of the refrigerator or controllers of the other refrigerator, and is used to regulate an operating state of the deodorization and sterilization apparatus according to the operation condition of the refrigerator), so that the boost circuit 4132 performs conversion to obtain the first voltage or the second voltage according to the control signal. The inverter is configured to invert the direct current power supply 4130 into alternating current in a controlled manner. A voltage of the alternating current is the first voltage or the second voltage. An effective value of the first voltage ranges from 2000 to 5000 V. An effective value of the second voltage ranges from 1000 to 1800 V. A length of the connecting cable 430 is in a range of less than 100 cm, and preferably in a range of 10 cm to 20 cm.

The deodorization and sterilization apparatus 400 may also be connected to a current detection device. The current detection device is configured to detect a current value provided to the power supply input interface 4131, so as to determine the operating state of the deodorization and sterilization apparatus. The current detection device may be connected in series to a power supply wire, which supplies electricity to the power supply input interface 4131. By using the current detection device to detect current, whether the deodorization and sterilization apparatus operates normally or not may be determined.

It should be understood by those skilled in the art that the "air-cooled refrigerator" referred to in the present invention is not limited to the air-cooled refrigerator provided with a refrigeration chamber and a freezing chamber and used to store food in a general sense, and may also be other devices with refrigeration functions, such as wine cabinets, refrigeration tanks, etc.

In the air-cooled refrigerator according to the embodiment of the present invention, through bombardment and excitation by high-energy electrons generated through high-voltage discharging, high-energy and highly-activity free radicals such as atomic oxygen (O) and hydroxyl (OH⁻) may be generated. The high-energy high-activity free radicals collide with odor gas molecules directly and frequently. When the energy obtained by the odor gas molecules is greater than binding energy of the molecular bond energy, original molecular structures of the odor gas molecules are damaged, molecular chemical bonds are opened to promote fast proceeding of a gaseous reaction, and atomic groups and solid particles are generated. Additionally, during high-voltage discharging, parts of odor gas molecules may also be ionized and decomposed. Under the action of the above principle, the deodorization and sterilization apparatus 400 is disposed in the air duct, and all of the free radicals are brought to respective compartments of the refrigerator through air path circulation, so as to achieve the effect that the free radicals are spread over the respective compartments of the entire refrigerator, thereby deodorizing and sterilizing the respective compartments of the refrigerator. Additionally, through effective placement modes and sealing measures, water vapor is prevented from condensing on the control module 410 of the deodorization and sterilization apparatus 400, and at the same time, even if condensation accumulation occurs, this condensed water may also be drained away.

## Claims

1. An air-cooled refrigerator, comprising a cabinet (200), an evaporator (214) and an air duct assembly (300), wherein a storage space (210) is defined inside the cabinet (200); the evaporator (214) and the air duct assembly (300) are disposed inside the cabinet (200); and air supplied to the storage space (210) is cooled by the evaporator (214); the air duct assembly (300) is provided with at least one air supply duct (310), and is configured to supply the air cooled by the evaporator (214) to the storage space (210) in a controlled manner; wherein
an accommodation space (320) is disposed inside the cabinet (200) or the air duct assembly (300), and the accommodation space (320) is located outside each of the at least one air supply duct (310); and
the air-cooled refrigerator further comprises a deodorization and sterilization apparatus (400), and the deodorization and sterilization apparatus (400) is provided with:
a control module (410), disposed in the accommodation space (320);
an ion generation module (420), disposed inside one of the at least one air supply duct (310); and
a connecting cable (430), electrically connected to the control module (410) and the ion generation module (420),
wherein the ion generation module (420) includes an ionization head and a base, wherein the ionization head is provided with a first electrode and a second electrode connected with the connecting cable (430) and wherein a discharging gap is formed between the first electrode and the second electrode, and the discharging gap is configured to be broken down by a first voltage so that the surrounding air is excited to be ionized, wherein
the control module (410) is provided with a control circuit module (413), and the control circuit module (413) comprises a power supply input interface (4131), a boost circuit (4132) and a high-voltage output interface (4133); the power supply input interface (4131) is configured to be connected with a direct current power supply (4130) provided externally; the boost circuit (4132) is configured to convert the direct current power supply (4130) into the first voltage for generating a deodorization and sterilization substance and a second voltage for heating in a controlled manner; and the high-voltage output interface (4133) is configured to output the first voltage or the second voltage, wherein the first voltage is higher than the second voltage;
the connecting cable (430) is connected to the high-voltage output interface (4133) and the ion generation module (420); and
the ion generation module (420) is configured to be excited by the first voltage to ionize air so as to generate the deodorization and sterilization substance, and to generate heat at the second voltage so as to eliminate condensation.

2. The air-cooled refrigerator according to claim 1, wherein
the deodorization and sterilization substance generated by the ion generation module (420) comprises at least one of singlet active oxygen, oxygen anion, hydroxyl radical, ozone and hydrogen peroxide.

3. The air-cooled refrigerator according to claim 1, wherein
the at least one air supply duct (310) comprises a first air duct (311) configured to supply air to an upper portion of the storage space (210);
a lower portion of the storage space (210) is provided with an air return port (220); and
the ion generation module (420) is disposed in the first air duct (311).

4. The air-cooled refrigerator according to claim 3, wherein
the air duct assembly (300) further comprises an air duct foam (340) and an air duct cover plate (350);
at least one of the at least one air supply duct (310) is defined at a back side of the air duct foam (340);
the accommodation space (320) and a cable groove (3110) connecting the accommodation space (320) and the first air duct (311) are disposed in a front side of the air duct foam (340); the connecting cable (430) is installed in the cable groove (3110); and
the air duct cover plate (350) is installed at the front side of the air duct foam (340).

## Patentansprüche

1. Luftgekühlter Kühlschrank, umfassend ein Gehäuse (200), einen Verdampfer (214) und eine Luftkanalanordnung (300), wobei ein Aufbewahrungsraum (210) innerhalb des Gehäuses (200) definiert ist; der Verdampfer (214) und die Luftkanalanordnung (300) innerhalb des Gehäuses (200) angeordnet sind; und dem Aufbewahrungsraum (210) zugeführte Luft durch den Verdampfer (214) gekühlt wird; die Luftkanalanordnung (300) mit zumindest einem Luftzufuhrkanal (310) vorgesehen ist, und konfiguriert ist, um die durch den Verdampfer (214) gekühlte Luft auf eine kontrollierte Weise dem Aufbewahrungsraum (210) zuzuführen; wobei
ein Aufnahmeraum (320) innerhalb des Gehäuses (200) oder der Luftkanalanordnung (300) angeordnet ist, und der Aufnahmeraum (320) außerhalb jedes der zumindest einen Luftzufuhrkanäle (310) angeordnet ist; und
der luftgekühlte Kühlschrank des Weiteren eine Desodorierungs- und Sterilisationsvorrichtung (400) umfasst, und die Desodorierungs- und Sterilisationsvorrichtung (400) vorgesehen ist mit:
einem Steuermodul (410), das in dem Aufnahmeraum (320) angeordnet ist;
einem Ionenerzeugungsmodul (420), das in einem der zumindest einen Luftzufuhrkanäle (310) angeordnet ist; und
einem Verbindungskabel (430), elektrisch verbunden mit dem Steuermodul (410) und dem Ionenerzeugungsmodul (420),
wobei das Ionenerzeugungsmodul (420) einen Ionisationskopf und eine Basis umfasst, wobei der Ionisationskopf mit einer ersten Elektrode und einer zweiten Elektrode vorgesehen ist, die mit dem Verbindungskabel (430) verbunden sind, und wobei zwischen der ersten Elektrode und der zweiten Elektrode ein Entladungsspalt gebildet ist, und der Entladungsspalt konfiguriert ist, um durch eine erste Spannung durchbrochen zu werden, sodass die umgebende Luft angeregt wird, ionisiert zu werden, wobei
das Steuermodul (410) mit einem Steuerschaltungsmodul (413) vorgesehen ist, und das Steuerschaltungsmodul (413) eine Energieversorgungseingangsschnittstelle (4131), eine Boost-Schaltung (4132) und eine Hochspannungsausgabeschnittstelle (4133) umfasst; die Energieversorgungseingangsschnittstelle (4131) konfiguriert ist, um mit einer extern bereitgestellten Gleichstromenergieversorgung (4130) verbunden zu werden; die Boost-Schaltung (4132) konfiguriert ist, um die Gleichstromenergieversorgung (4130) in die erste Spannung zum Erzeugen einer desodorierenden und sterilisierenden Substanz und eine zweite Spannung zum Erwärmen auf kontrollierte Weise umzuwandeln; und die Hochspannungsausgabeschnittstelle (4133) konfiguriert ist, um die erste Spannung oder die zweite Spannung auszugeben, wobei die erste Spannung höher als die zweite Spannung ist;
das Verbindungskabel (430) mit der Hochspannungsausgabeschnittstelle (4133) und dem Ionenerzeugungsmodul (420) verbunden ist; und
das Ionenerzeugungsmodul (420) konfiguriert ist, um durch die erste Spannung erregt zu werden, um Luft zu ionisieren, um so die desodorierende und sterilisierende Substanz zu erzeugen, und um bei der zweiten Spannung Wärme zu erzeugen, um so Kondensation zu beseitigen.

2. Luftgekühlter Kühlschrank nach Anspruch 1, wobei
die von dem Ionenerzeugungsmodul (420) erzeugte Desodorisierungs- und Sterilisierungssubstanz zumindest eines von Singulett-Aktivsauerstoff, Sauerstoffanion, Hydroxylradikal, Ozon und Wasserstoffperoxid umfasst.

3. Luftgekühlter Kühlschrank nach Anspruch 1, wobei
der zumindest eine Luftzufuhrkanal (310) einen ersten Luftkanal (311) umfasst, der konfiguriert ist, um einem oberen Abschnitt des Aufbewahrungsraums (210) Luft zuzuführen;
ein unterer Abschnitt des Aufbewahrungsraums (210) mit einer Luftrückfuhröffnung (220) vorgesehen ist; und
das Ionenerzeugungsmodul (420) in dem ersten Luftkanal (311) angeordnet ist.

4. Luftgekühlter Kühlschrank nach Anspruch 3, wobei
die Luftkanalanordnung (300) des Weiteren einen Luftkanalschaum (340) und eine Luftkanalabdeckplatte (350) umfasst;
zumindest einer der zumindest einen Luftzufuhrkanäle (310) an einer Rückseite des Luftkanalschaums (340) definiert ist;
der Aufnahmeraum (320) und eine Kabelnut (3110), die den Aufnahmeraum (320) und den ersten Luftkanal (311) verbindet, in einer Vorderseite des Luftkanalschaums (340) angeordnet sind; das Verbindungskabel (430) in der Kabelnut (3110) installiert ist; und
die Luftkanalabdeckplatte (350) an der Vorderseite des Luftkanalschaums (340) installiert ist.

## Revendications

1. Un réfrigérateur à refroidissement par air, comprenant une armoire (200), un évaporateur (214) et un conduit d'aération (300), dans lequel un espace de stockage (210) est défini à l'intérieur
de l'armoire (200), l'évaporateur (214) et le conduit d'aération (300) sont disposés à l'intérieur de l'armoire (200); et l'air fourni à l'espace de stockage (210) est refroidi par l'évaporateur (214); le conduit d'aération (300) est pourvu d'au moins un conduit d'alimentation d'air (310) et est configuré pour fournir l'air refroidi par l'évaporateur (214) à l'espace de stockage (210) de manière contrôlée ;
un espace de logement (320) est disposé à l'intérieur de l'armoire (200) ou le conduit d'aération (300) et l'espace de logement (320) est situé à l'extérieur de chacun du ou des conduits d'alimentation d'air (310); et
le réfrigérateur à refroidissement par air comprend en outre un appareil de désodorisation et de stérilisation (400), et l'appareil de désodorisation et de stérilisation (400) est
pourvu :
d'un module de commande (410), disposé dans l'espace de logement (320);
un module de génération d'ions (420), disposé à l'intérieur du ou des conduits d'alimentation d'air (310); et
un câble de connexion (430), connecté électriquement au module de commande (410)
et le module de génération d'ions (420),
dans lequel le module de génération d'ions (420) comprend une tête d'ionisation et une base, dans lequel la tête d'ionisation est pourvue d'une première électrode et d'une seconde électrode reliée au câble de connexion (430) et dans lequel un espace de décharge se forme entre la première électrode et la seconde électrode,
et l'espace de décharge est configuré pour être décomposé par une première tension de telle sorte que l'air ambiant est agité afin d'être ionisé, dans lequel
le module de commande (410) est pourvu d'un module de circuit de commande (413) et le module de circuit de commande (413) comprend une interface d'entrée d'alimentation électrique (4131), un circuit de suralimentation (4132) et une interface de sortie haute tension (4133);
l'interface d'entrée d'alimentation électrique (4131) est configurée pour être connectée avec une alimentation électrique en courant (4130) fournie à l'extérieur; le circuit de suralimentation (4132) est configuré pour convertir l'alimentation électrique en courant (4130) en première tension pour générer une substance de désodorisation et de stérilisation et une deuxième tension pour un chauffage de manière contrôlée; et l'interface de sortie haute tension (4133) est configuré pour générer la première tension ou la deuxième tension, dans lequel la première la tension est supérieure à la deuxième tension;
le câble de connexion (430) est connecté à l'interface de sortie haute tension (4133) et au module de génération d'ions (420); et
le module de génération d'ions (420) est configuré pour être agité par la première tension pour ioniser l'air afin de produire la substance de désodorisation et la stérilisation, et de générer de la chaleur lors de la deuxième tension afin d'éliminer la condensation.

2. Le réfrigérateur à refroidissement par air selon la revendication 1,
dans lequel la substance de désodorisation et de stérilisation générée par le module de génération d'ions (420) comprend au moins un produit tel que l'oxygène actif singulet, l'ion d'oxygène, le radical hydroxyle, l'ozone et le peroxyde d'hydrogène.

3. Le réfrigérateur à refroidissement par air selon la revendication 1,
dans lequel au moins un conduit d'alimentation d'air (310) comprend un premier conduit d'air (311) configuré pour fournir de l'air à une partie supérieure de l'espace de stockage (210);
une partie inférieure de l'espace stockage (210) est pourvue d'un orifice de retour d'air (220); et
le module de génération d'ions (420) est disposé dans le premier conduit d'air (311).

4. Le réfrigérateur à refroidissement par air selon la revendication 3,
dans lequel le conduit d'aération (300) comprend en outre une mousse de conduit d'air (340) et une plaque de couverture de conduit d'air (350);
au moins un du ou des conduits d'alimentation d'air (310) est défini sur un côté arrière
de la mousse de conduit d'air (340);
l'espace de logement (320) et une rainure de câble (3110) reliant l'espace de logement (320) et le premier conduit d'air (311) sont disposés sur un côté avant de la mousse de conduit d'air (340); le câble de connexion (430) est installé dans la rainure du câble (3110); et
la plaque de couverture du conduit d'air (350) est installée sur un côté avant de la mousse de conduit d'air (340).
